# EUROPEAN PATENT APPLICATION

(11) **EP 4 497 476 A1**
(43) Date of publication of application: **29.01.2025**
(21) Application number: 23774912.2
(22) Date of filing: 22.03.2023
(51) Int. Cl.: A61P 35/00, C07H 23/00, C08G 65/32, C08G 65/337, A61K 41/00, A61K 31/69, A61K 31/724

(54) **BORONIC ACID-CONTAINING MODIFIED POLYROTAXANE**

(30) Priority: 23.03.2022 JP 2022046218
(71) Applicant: National University Corporation Kumamoto University, Kumamoto-shi, Kumamoto 860-8555 (JP); University of Tsukuba, Tsukuba-shi, Ibaraki 305-8577 (JP)
(72) Inventor: HIGASHI Taishi, Kumamoto-shi, Kumamoto 860-8555 (JP); MOTOYAMA Keiichi, Kumamoto-shi, Kumamoto 860-8550 (JP); ONODERA Risako, Kumamoto-shi, Kumamoto 860-8555 (JP); MATSUMOTO Yoshitaka, Tsukuba-shi, Ibaraki 305-8550 (JP)
(74) Representative: Maiwald GmbH
(86) International application number: PCT/JP2023/011056
(87) International publication number: WO 2023/182317

(57) **Abstract**

The present invention aims to provide a novel compound that can be a component of boron drugs. The present invention provides a boronic acid-containing modified polyrotaxane, which is a polyrotaxane having a plurality of cyclodextrin molecules, a linear molecule that pierces the openings of the plurality of cyclodextrin molecules in a skewered manner, and blocking groups that are arranged at both ends of the linear molecule and prevent the cyclodextrin molecule from being separated from the linear molecule, in which at least a portion of the cyclodextrin molecules is bonded via a linker to a monovalent group derived from boronic acid or a derivative thereof.

## Description

### [Technical Field]

The present invention relates to a modified polyrotaxane containing boronic acid, in particular to a modified polyrotaxane that is a novel boronic acid-containing compound that can be used in boron neutron capture therapy. The present invention also relates to a pharmaceutical composition containing the boronic acid-containing modified polyrotaxane.

### [Background Art]

In recent years, boron neutron capture therapy (BNCT) has attracted much attention as a new cancer treatment method. BNCT is a non-invasive and effective radiation therapy in which a drug containing boron (¹⁰B) is accumulated in the cancer, and then neutron radiation is applied to damage only the cancer cells with radiation generated by the nuclear fission of boron. Currently, known boron drugs include the first generation, mercaptoundecahydrododecaborate (BSH), a boron cluster, and the second generation, p-boronophenylalanine (BPA), in which one boron atom is bound to phenylalanine. In addition, third generation boron drugs in which boron clusters are loaded onto carriers such as liposomes, polymers, polymer micelles, and the like have also been reported.

BSH is a boron drug having an icosahedral three-dimensional molecule containing 12 boron atoms in one molecule, and is a drug with excellent water solubility, but has no intracellular uptake ability and has poor selectivity to tumors. In the third generation boron drug including BSH, boron can be selectively delivered to tumors by the Enhanced Permeability and Retention effect (EPR effect) by using a carrier such as a liposome, polymer, or polymer micelle. However, since the EPR effect is generally realized by a carrier that shows long-term blood retention, it is difficult to increase the tumor/blood boron concentration ratio when using a third generation boron drug.

BPA is the most widely used, and it is taken up into cancer cells via amino acid transporters that are highly expressed on the surface of cancer cells. However, BPA is not only poorly water-soluble, but also has a low molecular weight, so it is quickly excreted from the body. Even if it reaches cancer cells, it is quickly excreted from the cancer cells via an extracellular excretion mechanism. Therefore, there is a problem that it has low blood retention and cancer accumulation. As a result, very high doses and continuous administration (about 30 g is administered intravenously over 2 hours, and administration is continued during neutron irradiation) are required to maintain the boron concentration in the tumor, and adverse events due to high doses have also been reported. Therefore, a boron drug that accumulates in cancers with high selectivity and is easy to administer (for example, can be administered in a single dose) is desired.

For a drug delivery system (DDS) that improves the accumulation of boron drugs in cancer, research has been reported on reversibly binding BPA to polyvinyl alcohol and polymerizing it (Patent Document 1, Non-Patent Document 1). By polymerizing it, it can accumulate in cancer tissues via the EPR effect (passive targeting) and avoid extracellular excretion by transporters. This method is a very practical attempt because it can easily improve the cancer accumulation of BPA, but it is a DDS that mainly relies on passive targeting and has a limited cancer accumulation (about 5% of the dose).

In addition, it has been reported that encapsulating a boron drug in a PEGylated liposome improves retention in the tumor, and the BPA concentration in the tumor can be maintained at 30 µg/g tissue for at least 72 hours after intravenous administration (Non-Patent Document 2).

In general, in BNCT, in order to reduce damage to normal tissues, it is required that the boron concentration in the tumor is 20 to 40 µg ¹⁰B/g tissue, and that the tumor/blood ratio (T/B ratio) or tumor/normal tissue ratio (T/N ratio) is 3 or more. Therefore, a boron drug that can meet these criteria is desired.

Known methods for imparting cancer cell and tumor tissue selectivity to carriers include ligand modification with small molecules such as antibodies, peptides, sugars, vitamins, phenylboronic acid (PBA) and the like. For example, Tang et al. reported that selective delivery to cancer cells is possible by modifying nanoparticles with PBA (Non-Patent Document 3). Deshayes et al. reported that nanocarriers in which polymeric micelles are modified with PBA is significantly improved in uptake by cancer cells after intravenous administration compared to non-PBA modified groups, and the therapeutic effect is enhanced (Non-Patent Document 4).

Cyclodextrins (CyD) are cyclic oligosaccharides, classified as monomolecular host molecules that incorporate various drugs into its hydrophobic cavity to form inclusion complexes, and are used in many fields, including foods, cosmetics, clinical diagnostics, membrane science, and polymer chemistry. In recent years, research has been actively conducted into the effective use of CyD supramolecules as biomaterials and drug carriers. It has been reported that α-CyD forms a necklace-shaped inclusion complex, polypseudorotaxane (PPRX), with polyethylene glycol (PEG). It has also been reported that β- CyD, which has a large cavity diameter, selectively forms PPRX with polypropylene glycol (PPG). Furthermore, it has been reported that the introduction of a bulky substituent to the end of the axis molecular polymer in PPRX results in a polyrotaxane (PRX) in which CyD is trapped within the molecule (Non-Patent Documents 5-6). In the pharmaceutical field, PPRX and PRX have been developed in which receptor-recognition sugars such as galactose and maltose are bound to CyD molecules, and it has been reported that the ligands are mobile and capable of multivalent interactions with lectins (Non-Patent Documents 7-9).

### [Cited References]

### [Patent Document]

### [Patent Document 1] WO2019/163790

### [Non-Patent Document]

[Non-Patent Document 1] Nomoto et. al., Poly(vinyl alcohol) boosting therapeutic potential of p-boronophenylalanine in neutron capture therapy by modulating metabolism. Sci. Adv., 6, eaaz1722 (2020)
[Non-Patent Document 2] Maruyama et al., Intracellular targeting of sodium mercaptoundecahydrododecaborate (BSH) to solid tumors by transferrin-PEG liposomes, for boron neutron-capture therapy (BNCT). J. Control. Release, 98, 195-207 (2004)
[Non-Patent Document 3] Tang et al., Cell-Selective Messenger RNA Delivery and CRISPR/Cas9 Genome Editing by Modulating the Interface of Phenylboronic Acid-Derived Lipid Nanoparticles and Cellular Surface Sialic Acid. ACS Appl. Mater. Interfaces, 11, 46585-46590 (2019)
[Non-Patent Document 4] Deshayes et. al., Phenylboronic acid-installed polymeric micelles for targeting sialylated epitopes in solid tumors. J. Am. Chem. Soc., 135, 15501-15507 (2013)
[Non-Patent Document 5] Harada et. al., The molecular necklace: a rotaxane containing many threaded a-cyclodextrins. Nature, 356, 325-327 (1992)
[Non-Patent Document 6] Jiao et. al., Inclusion complexes of single-C60-end-capped poly (ethylene oxide) with cyclodextrins. Macromolecules, 35, 1399-1402 (2002)
[Non-Patent Document 7] Nelson et. al., A self-assembled multivalent pseudopolyrotaxane for binding galectin-1. J. Am. Chem. Soc., 126, 11914-11922 (2004)
[Non-Patent Document 8] Ooya et. al., Supramolecular design for multivalent interaction: maltose mobility along polyrotaxane enhanced binding with concanavalin A. J. Am. Chem. Soc., 125, 13016-13017 (2003)
[Non-Patent Document 9] Ooya et. al., Effects of polyrotaxane structure on polyion complexation with DNA. Sci. Tech. Adv. Mater., 5, 363 (2004)

### [Technical Problem]

An object of the present invention is to provide novel compounds which can be components of boron drugs.

### [Solution to Problem]

The present inventors have conducted research to provide a boron drug that is improved in at least one, preferably in more than one, of blood retention, tumor selectivity, and tumor/blood boron concentration ratio, compared to conventional boron drugs. In the process, the present inventors came up with an idea that phenylboronic acid (PBA) itself can be a boron drug, and therefore can serve as both a carrier and a drug in BNTC, and focused on PBA. PBA is preferable as a ligand molecule because of the following advantages: 1) it targets sialic acid (SA), a tumor marker used in clinical cancer diagnosis; 2) it has a relatively small molecular weight of 184 Da, which does not affect the pharmacokinetics of the carrier; and 3) it is inexpensive. As a result of intensive research, the present inventors have found that modified polyrotaxanes containing boronic acid, including PBA, exhibit superior effects compared to conventional boron drugs, and have completed the present invention.

The present invention includes the following.
[1] A boronic acid-containing modified polyrotaxane comprising a plurality of cyclodextrin molecules, a linear molecule that pierces the openings of the plurality of cyclodextrin molecules in a skewered manner, and blocking groups that are positioned at both ends of the linear molecule and prevent separation of the cyclodextrin molecule from the linear molecule, wherein at least a portion of the cyclodextrin molecules are bonded via a linker to a monovalent group derived from boronic acid or a derivative thereof.
[2] The boronic acid-containing modified polyrotaxane according to [1] above, wherein the monovalent group derived from boronic acid or a derivative thereof is a monovalent group derived from a phenylboronic acid derivative or a pyridineboronic acid derivative.
[3] The boronic acid-containing modified polyrotaxane according to [2] above, wherein the monovalent group derived from boronic acid or a derivative thereof is a group represented by the following formula (B1): 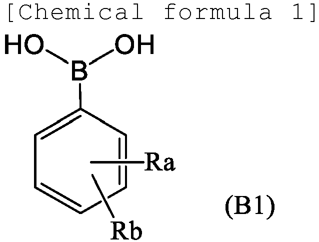 (wherein Ra represents a hydrogen atom or a halogen atom, and Rb represents -(CH₂)n-CO-*, -(CH₂)n-NH-*, - (CH₂)n-CH(NHR)CO-*, or -(CH₂)n-CH(C(O)OR)NH-* (wherein * represents a bond to a linker, n represents 0, 1, or 2, and R represents a hydrogen atom or a C1-C5 hydrocarbon group which may have a substituent)), or the following formula (B2): 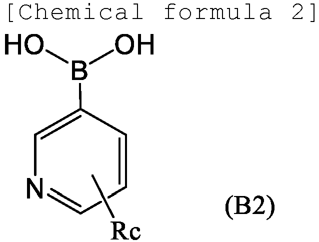 (wherein Rc represents -(CH₂)n-CO-*, -(CH₂)n-NH-*, - (CH₂)n-CH(NHR)CO-*, or -(CH₂)n-CH(C(O)OR)NH-* (wherein * represents a bond to a linker, n represents 0, 1, or 2, and R represents a hydrogen atom or a C1-C5 hydrocarbon group which may have a substituent)).
[4] The boronic acid-containing modified polyrotaxane according to [3] above, wherein the monovalent group derived from boronic acid or a derivative thereof is a group represented by the following formula (B3): 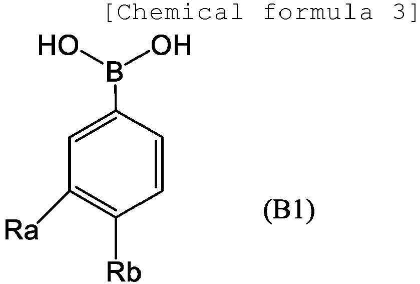 (wherein Ra represents a hydrogen atom or a halogen atom, and Rb represents -(CH₂)n-CO-*, -(CH₂)n-NH-*, - (CH₂)n-CH(NH₂)CO-*, or -(CH₂)n-CH(C(O)OH)NH-* (wherein * represents a bond to a linker, and n represents 0 or 1)), or the following formula (B4): 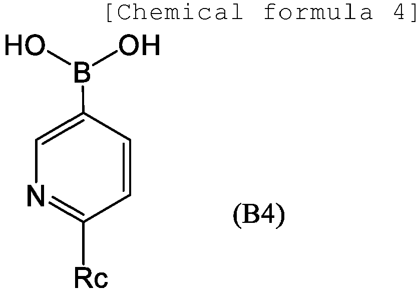 (wherein Rc represents -(CH₂)n-CO-*, -(CH₂) n-NH-*, - (CH₂)n-CH(NH₂)CO-*, or -(CH₂)n-CH(C(O)OH)NH-* (wherein * represents a bond to a linker, and n represents 0 or 1)) .
[5] The boronic acid-containing modified polyrotaxane according to [4] above, wherein the monovalent group derived from boronic acid or a derivative thereof is a monovalent group derived from a boronic acid derivative selected from the group consisting of 4-carboxyl-3-fluorophenylboronic acid, 6-carboxypyridine-3-boronic acid (5-boronopicolinic acid), 4-carboxyphenylboronic acid, p-boronophenylalanine, 4-amino-3-fluorophenylboronic acid, and 5-aminopyridine-3-boronic acid.
[6] The boronic acid-containing modified polyrotaxane according to any one of [1] to [5] above, wherein the linear molecule is polyethylene glycol, polypropylene glycol, or a copolymer of polyethylene glycol and polypropylene glycol.
[7] The boronic acid-containing modified polyrotaxane according to any one of [1] to [6] above, wherein the blocking group is an adamantyl group.
[8] The boronic acid-containing modified polyrotaxane according to any one of [1] to [7] above, wherein the plurality of cyclodextrin molecules are a plurality of α-cyclodextrin molecules.
[9] The boronic acid-containing modified polyrotaxane according to any one of [1] to [8] above, wherein the linker is represented by the formula (L1): 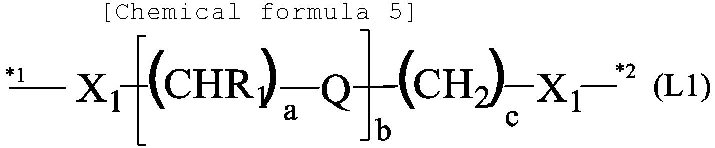 [wherein, *¹ is a site bonding to a cyclodextrin molecule, *² is a site bonding to a group containing boronic acid,
   R₁ each independently represents a hydrogen atom, a carboxy group which may be substituted with an alkyl group having 1 to 6 carbon atoms, a hydroxy group which may be substituted with an alkyl group having 1 to 6 carbon atoms, a mercapto group which may be substituted with an alkyl group having 1 to 6 carbon atoms, an aryl group having 6 to 14 carbon atoms, or an alkyl group having 1 to 6 carbon atoms;
   Q each independently represents -O-, -S-, -NH-, - SO₂-, -CO-, -NHCO-, -CONH-, -OCO-, -COO-, -SCO-, -COS-, -NHCONH-, -NHCSNH-, -OCONH-, or -NHCOO-;
   a each independently represents an integer of 1 to 6;
   b represents an integer of 1 to 6;
   c represents an integer of 1 to 6;
   X₁ each independently represents -O-, -S-, -NH-, - SO₂-, -CO-, -NHCO-, -CONH-, -OCO-, -COO-, -SCO-, -COS-, -NHCONH-, -NHCSNH-, -OCONH-, or -NHCOO-].
[10] The boronic acid-containing modified polyrotaxane according to [9] above, wherein the linker is represented by the formula (L2): 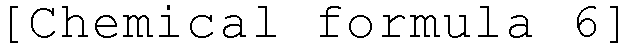 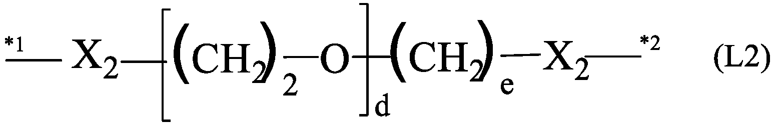 [wherein, *¹ is a site bonding to a cyclodextrin molecule, *² is a site bonding to a group containing boronic acid,
   d represents an integer of 1 to 6;
   e represents an integer of 1 to 3;
   X₂ each independently represents -O-, -S-, -NH-, - SO₂-, -CO-, -NHCO-, -CONH-, -OCO-, -COO-, -SCO-, -COS-, -NHCONH-, -NHCSNH-, -OCONH-, or -NHCOO-].
[11] The boronic acid-containing modified polyrotaxane according to any one of [1] to [10] above, wherein in the polyrotaxane in which the blocking group is an adamantyl group, folate-modified β-cyclodextrin is further bonded to the adamantyl group.
[12] The boronic acid-containing modified polyrotaxane according to [11], wherein the plurality of cyclodextrin molecules are α-cyclodextrin molecules and the linear molecule is polyethylene glycol.
[13] The boronic acid-containing modified polyrotaxane according to any one of [1] to [12] above, wherein polyethylene glycol-modified dopamine is further bonded to the boronic acid.
[14] A pharmaceutical composition for use in boron neutron capture therapy for tumor diseases, comprising the boronic acid-containing modified polyrotaxane according to any one of [1] to [13] in which the boron in the boronic acid is ¹⁰B.
[15] A method for treating a tumor disease by boron neutron capture therapy, comprising administering to a mammal a required amount of the boronic acid-containing modified polyrotaxane according to any one of [1] to [13] in which the boron in the boronic acid is ¹⁰B.

### [Advantageous Effect of the Invention]

The present invention provides a novel compound containing boron. The boronic acid-containing polyrotaxane provided by the present invention can be used as a boron drug for boron neutron capture therapy.

### [Brief Description of Drawings]

[Figure 1]

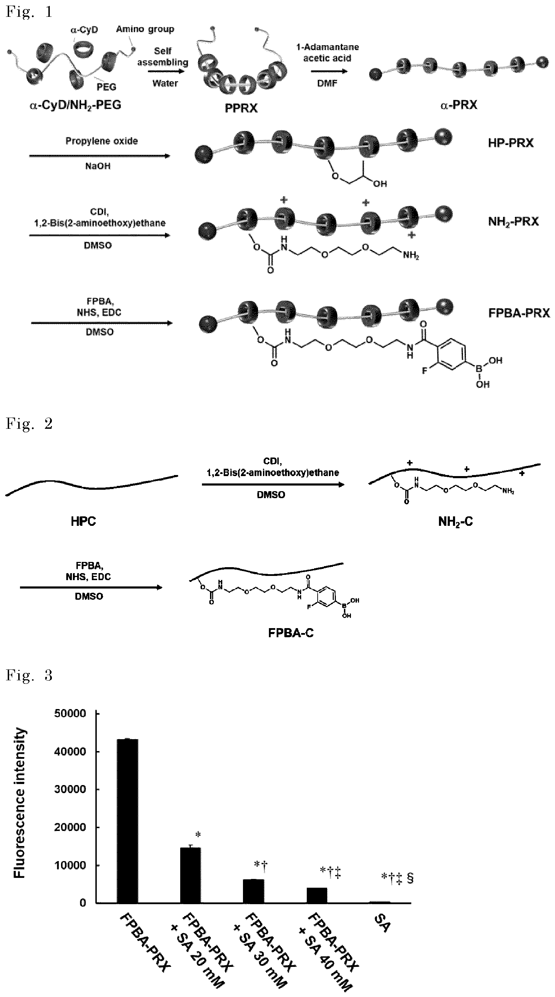

   FIG. 1 shows the preparation route of FPBA-PRXX (fluorophenylboronic acid-rotaxane) in Example 1.
[Figure 2]
   FIG. 2 shows the preparation route of FPBA-C (fluorophenylboronic acid-cellulose) in Comparative Example 1.
[Figure 3]
   FIG. 3 shows the results of confirming the binding of Alizarin Red S (ARS) to FPBA-PRX and the inhibitory effect of sialic acid (SA). Each value represents the mean value ± SEM of six measurements. * indicates P<0.05 compared to FPBA-PRX, † indicates P<0.05 compared to FPBA-PRX + SA 20 mM, ++ indicates P<0.05 compared to FPBA-PRX + SA 30 mM, and § indicates P<0.05 compared to FPBA-PRX + SA 40 mM.
[Figure 4]

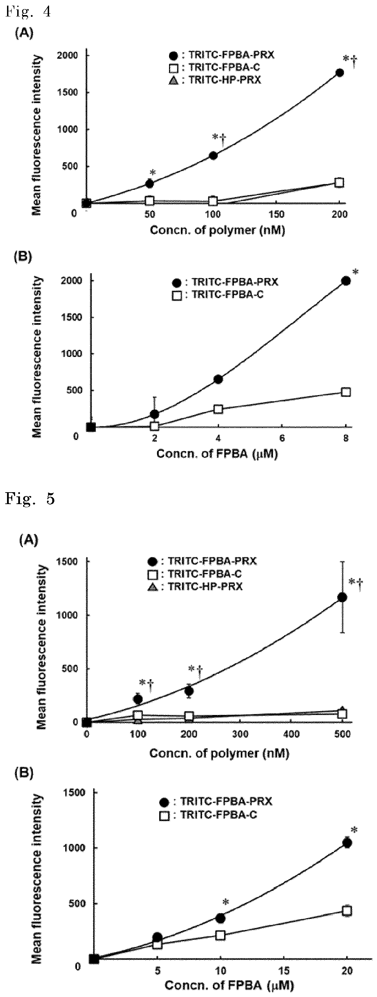

   FIG. 4A shows the results of measuring the uptake of TRITC-HP-PRX, TRITC-FPBA-PRX, and TRITC-FPBA-C into HeLa cells, based on the polymer concentration. * indicates P<0.05 compared to TRITC-HP-PRX, and † indicates P<0.05 compared to TRITC-FPBA-C. FIG. 4B shows the results of measuring the uptake of TRITC-FPBA-PRX and TRITC-FPBA-C into HeLa cells, based on the FPBA concentration. * indicates P<0.05 compared to TRITC-FPBA-C. Each value represents the mean value ± SEM of 3-4 measurements.
[Figure 5]
   FIG. 5A shows the results of measuring the uptake of TRITC-HP-PRX, TRITC-FPBA-PRX, and TRITC-FPBA-C into Colon-26 cells, based on the polymer concentration. * indicates P<0.05 compared to TRITC-HP-PRX, and † indicates P<0.05 compared to TRITC-FPBA-C. FIG. 5B shows the results of measuring the uptake of TRITC-FPBA-PRX and TRITC-FPBA-C into Colon-26 cells, based on the FPBA concentration. * indicates P<0.05 compared to TRITC-FPBA-C. Each value represents the mean value ± SEM of 3-4 measurements.
[Figure 6]

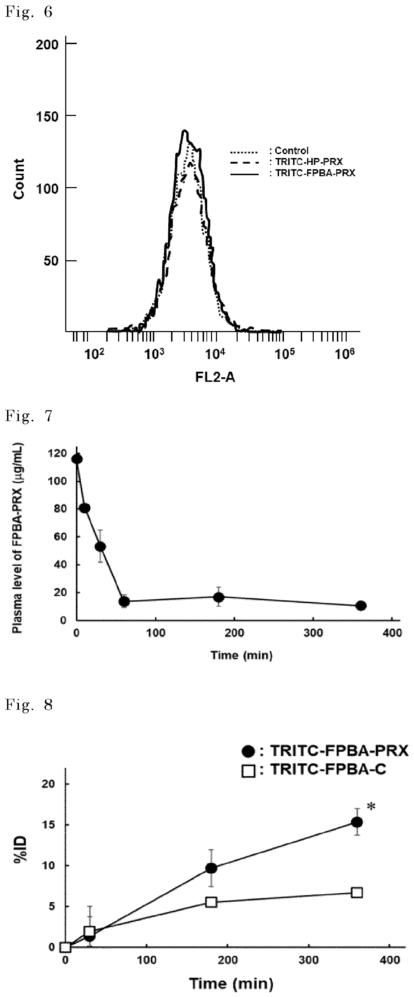

   FIG. 6 shows the results of the uptake of TRITC-HP-PRX and TRITC-FPBA-PRX into HK2 cells measured by flow cytometry. Each line shows representative data from 3-4 measurements.
[Figure 7]
   FIG. 7 shows the results of measuring the blood retention of TRITC-FPBA-PRX administered into the tail vein of mice. Each point represents the mean value ± SEM of six measurements.
[Figure 8]
   FIG. 8 shows the results of confirming the accumulation of TRITC-FPBA-PRX in tumors after administration into the tail vein of tumor-bearing mice. Each point represents the mean value ± SEM of 6-9 measurements. * indicates P<0.05 compared to TRITC-FPBA-C.
[Figure 9]

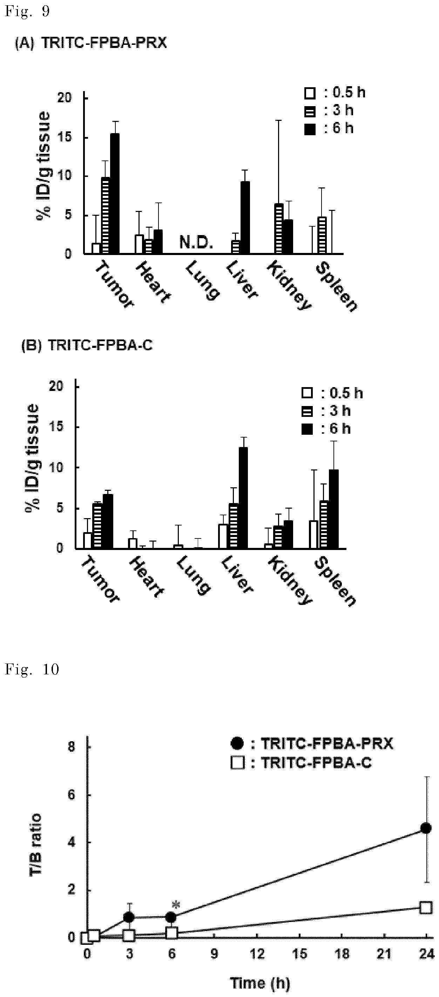

   FIG. 9 shows the results of confirming the accumulation of TRITC-FPBA-PRX and TRITC-FPBA-C in each tissue after administration into the tail vein of tumor-bearing mice. Each point represents the mean value ± SEM of six measurements. * indicates P<0.05 compared to TRITC-FPBA-C.
[Figure 10]
   FIG. 10 shows the results of measuring the T/B ratio of TRITC-FPBA-PRX and TRITC-FPBA-C administered into the tail vein of tumor-bearing mice. Each point represents the mean value ± SEM of 6-9 measurements. * indicates P<0.05 compared to TRITC-FPBA-C.
[Figure 11]

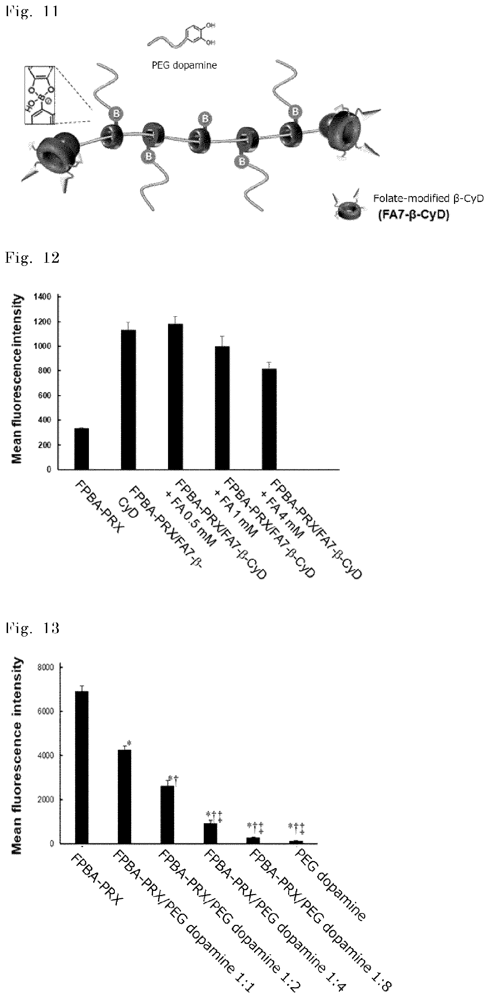

   FIG. 11 is a view schematically showing how folate-modified β-CyD or PEG-dopamine binds to the boronic acid-containing modified polyrotaxane of the present invention.
[Figure 12]
   FIG. 12 shows the results of evaluating the intracellular uptake of TRITC-FPBA-PRX/FA7-β-CyD. Each value represents the mean value ± SEM of three measurements.
[Figure 13]
   FIG. 13 shows the results of the evaluation of intracellular uptake of TRITC-FPBA-PRX/PEG-dopamine. Each value represents the mean value ± SEM of six measurements. * indicates P<0.05 compared to FPBA-PRX, † indicates P<0.05 compared to FPBA-PRX + PEG dopamine 220 µM, and ++ indicates P<0.05 compared to FPBA-PRX + PEG dopamine 440 µM TRITC-FPBA-C.
[Figure 14]

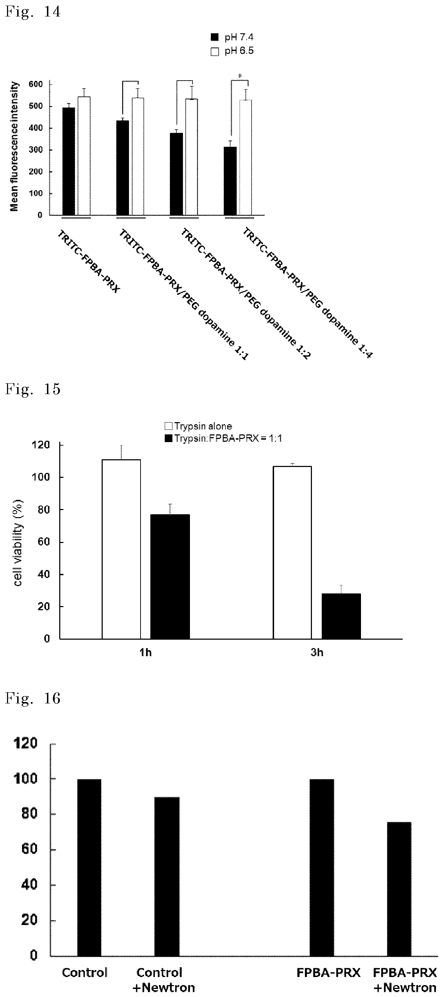

   FIG. 14 shows the results of confirming the effect of pH on TRITC-FPBA-PRX/PEG-dopamine. For each condition, the left column shows the results at pH 7.4, and the right column shows the results at pH 6.5. Each value represents the mean value ± SEM of 5-6 measurements. * indicates P<0.05 compared to the control.
[Figure 15]
   FIG. 15 shows the results of confirming the cytotoxicity of FPBA-PRX against cancer cells. At each time point, the left column shows the results for trypsin alone, and the right column shows the results for trypsin + FPBA-PRX. Each value represents the mean value ± SEM of four measurements. * indicates P<0.05 compared to the control (trypsin).
[Figure 16]
   FIG. 16 shows the results of confirming the antitumor effect of FPBA-PRX. The average tumor size of the neutron-unirradiated group is set as 100%, and the average tumor size of the irradiated group is shown.

### [Description of Embodiments]

The invention is described below by way of exemplary embodiments, along with preferred methods and materials that can be used in the practice of the invention. The subject matter of the present invention should not be limited to the embodiments described below. It should be noted that, unless otherwise specified herein, all technical and scientific terms used herein have the same meanings as commonly understood by those skilled in the art to which this invention pertains. Also, any materials and methods equivalent or similar to those described herein can also be used in the practice of the invention. Additionally, all publications and patents cited in the present specification in connection with the invention described in the present specification constitute a part of the present specification as indicating, for example, methods, materials, etc., that can be used in the present invention.

In the present specification, the notation "A-B" indicating a numerical range means a numerical range that indicates the endpoints A and B. The same applies to "A to B." Furthermore, in this specification, "about" is used to mean an allowance of ±10%.

### [Polyrotaxane]

The boronic acid-containing modified polyrotaxane of the present invention is composed of a plurality of cyclodextrin molecules, one linear molecule, and bulky blocking groups arranged at both ends of the linear molecule, and has a structure in which the linear molecule pierces the openings of the plurality of cyclodextrin molecules in a skewered manner, and the bulky blocking groups prevent the cyclodextrin molecule and the linear molecule from being separated. In the boronic acid-containing modified polyrotaxane of the present invention, at least a part of the cyclodextrin molecule is characterized in that it is bonded to a group containing boron via a linker. FIG. 1 shows a schematic view of the boronic acid-containing modified polyrotaxane. In the boronic acid-containing modified polyrotaxane of the present invention, it can exist as a boronic acid group R-B(OH)₂, so that it can bind to, for example, sialic acid (SA) that is highly expressed on the surface of cancer cells.

### [Cyclodextrin]

Examples of cyclodextrin molecules include α-cyclodextrin, β-cyclodextrin, and γ-cyclodextrin, and any of these cyclodextrins can be used in the present invention, but α-cyclodextrin or β-cyclodextrin is preferred, and α-cyclodextrin is more preferred. In the cyclodextrin molecule, a portion or all of the hydroxyl groups are bound to a group containing boronic acid via a linker.

### [Boronic acid or its derivative]

In the present invention, the boronic acid or a derivative thereof refers to a compound in which a hydroxy group of boric acid is substituted with an optionally substituted alkyl group having 1 to 6 carbon atoms (e.g., methyl group, ethyl group, propyl group, isopropyl group, butyl group, isobutyl group, sec-butyl group, tert-butyl group, pentyl group, hexyl group), an optionally substituted cycloalkyl group having 3 to 6 carbon atoms, an optionally substituted alkenyl group having 1 to 6 carbon atoms, an optionally substituted alkynyl group having 1 to 6 carbon atoms, an optionally substituted aryl group having 5 to 14 carbon atoms (e.g., phenyl group, naphthyl group, anthryl group, fluorenyl group), an optionally substituted 5 to 14 membered heteroaryl group (e.g., furyl group, benzofuryl group, pyridyl group, pyrimidinyl group, quinolyl group), an optionally substituted arylene group having 5 to 14 carbon atoms, or an optionally substituted 5 to 14 membered heteroarylene group, preferably a compound substituted with an optionally substituted phenyl group or an optionally substituted pyridyl group.

Examples of the substituent in the "optionally substituted" include halogen atoms (e.g., fluorine, chlorine, and bromine atoms), amino group, carboxy group, nitro group, cyano group, oxo group, hydroxy group, formyl group, carbamoyl group, thiocarbamoyl group, formylamino group, alkyl groups having 1 to 20 carbon atoms (e.g., methyl group, ethyl group, propyl group, isopropyl group, butyl group, isobutyl group, sec-butyl group, tert-butyl group, pentyl group, hexyl group, 2-ethylhexyl group, octyl group, dodecyl group, and dodecyloctyl group), aryl groups (e.g., phenyl group, naphthyl group, and fluorenyl group), aralkyl groups (e.g., benzyl group, and phenethyl group), and alkoxy groups (e.g., methoxy group, and ethoxy group). The number of the substituents in the "optionally substituted" is, for example, 1 to 3, and preferably 1 to 2. When there are two or more substituents, the respective substituents may be the same or different. The carbon atoms in these substituents are not included in the number of carbon atoms in the alkyl group or the like.

Other examples of the boronic acid or its derivative in the present invention include compounds described in WO2019/004482, JP2019-506915A, JP2019-503372A, and JP2019-513493A.

In the present invention, the boronic acid or its derivative includes preferably a phenylboronic acid derivative or a pyridineboronic acid derivative, and specific examples thereof include, but not limited thereto, 4-carboxyl-3-fluorophenylboronic acid, 6-carboxypyridine-3-boronic acid (5-boronopicolinic acid), 4- carboxyphenylboronic acid, p-boronophenylalanine, 4-amino-3-fluorophenylboronic acid, and 5-aminopyridine-3-boronic acid. The synthesis of the boronic acid derivative can be carried out with reference to a known method.

The monovalent group derived from boronic acid or a derivative thereof contained in the boronic acid-containing modified polyrotaxane of the present invention is a monovalent group derived from the above-mentioned boronic acid or derivative thereof, and examples thereof include groups represented by the following formula (B1): 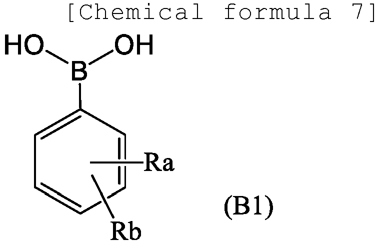 (wherein, Ra represents a hydrogen atom or a halogen atom, Rb represents -(CH₂)n-CO-*, -(CH₂)n-NH-*, -(CH₂)n-CH(NHR)CO-*, or -(CH₂)n-CH(C(O)OR)NH-* (wherein * represents a bond to a linker, n represents 0, 1, or 2, and R represents a hydrogen atom or a C1-C5 hydrocarbon group which may have a substituent)), and groups represented by the following formula (B2): 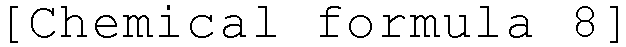 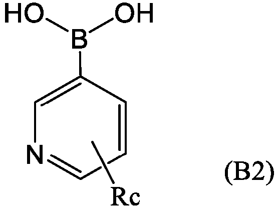 (wherein, Rc represents -(CH₂)n-CO-*, -(CH₂)n-NH-*, - (CH₂)n-CH(NHR)CO-*, or -(CH₂)n-CH(C(O)OR)NH-* (wherein * represents a bond to a linker, n represents 0, 1, or 2, and R represents a hydrogen atom or a C1-C5 hydrocarbon group which may have a substituent)).

Preferable examples thereof include groups represented by the following formula (B3): 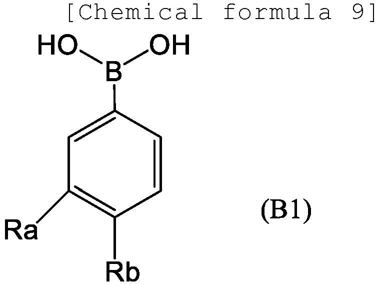 (wherein, Ra represents a hydrogen atom or a halogen atom, and Rb represents -(CH₂)n-CO-*, -(CH₂)n-NH-*, - (CH₂)n-CH(NH₂)CO-*, or -(CH₂)n-CH(C(O)OH)NH-* (wherein * represents a bond to a linker, and n represents 0 or 1)), and
groups represented by the following formula (B4): 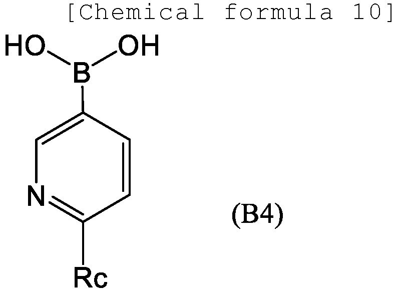 (wherein, Rc represents -(CH₂)n-CO-*, -(CH₂)n-NH-*, - (CH₂)n-CH(NH₂)CO-*, or -(CH₂)n-CH(C(O)OH)NH-* (wherein * represents a bond to a linker, and n represents 0 or 1)) .

In the above formula, examples of the halogen atom include fluorine, chlorine, bromine and iodine, with fluorine being preferred.

In the above formula, examples of the C1-C5 hydrocarbon group include methyl, ethyl, propyl, butyl, isobutyl, sec-butyl, tert-butyl, pentyl, isopentyl, and neopentyl.

Examples of the substituent in "which may have a substituent" in the above formula include halogen atoms (e.g., fluorine, chlorine, and bromine atoms), amino group, carboxy group, nitro group, cyano group, oxo group, hydroxy group, formyl group, carbamoyl group, thiocarbamoyl group, formylamino group, alkyl groups having 1 to 20 carbon atoms (e.g., methyl group, ethyl group, propyl group, isopropyl group, butyl group, isobutyl group, sec-butyl group, tert-butyl group, pentyl group, hexyl group, 2-ethylhexyl group, octyl group, dodecyl group, and dodecyloctyl group), aryl groups (e.g., phenyl group, naphthyl group, and fluorenyl group), aralkyl groups (e.g., benzyl group, and phenethyl group), and alkoxy groups (e.g., methoxy group, and ethoxy group). The number of the substituents in "which may have a substituent" is, for example, 1 to 3, and preferably 1 to 2. When there are two or more substituents, the respective substituents may be the same or different. The carbon atoms in these substituents are not included in the number of carbon atoms in the alkyl group or the like.

More preferred are monovalent groups derived from 4-carboxyl-3-fluorophenylboronic acid, 6-carboxypyridine-3-boronic acid (5-boronopicolinic acid), 4- carboxyphenylboronic acid, p-boronophenylalanine, 4-amino-3-fluorophenylboronic acid, or 5-aminopyridine-3-boronic acid.

In one embodiment of the present invention, a monovalent group derived from boronic acid or a derivative thereof that exhibits binding ability to sialic acid (SA) that is highly expressed on the surface of cancer cells is preferred, and 4-carboxyl-3-fluorophenylboronic acid and 6-carboxypyridine-3-boronic acid are particularly preferred because they exhibit strong binding ability to SA.

### [Linker]

The monovalent group derived from the boronic acid or its derivative is bonded to a part or all of the cyclodextrin molecule via a linker. The introduction of the linker into the cyclodextrin can be carried out, for example, by the following method, but is not limited thereto. A part or all of the hydroxyl groups of the cyclodextrin are hydroxypropylated with propylene oxide, and then a compound to be a linker is added. If necessary, a catalyst may be added to promote the reaction. This results in a polyrotaxane in which a compound to be a linker having a reactive group at one end is introduced into the cyclodextrin via a hydroxyl group of cyclodextrin and/or a hydroxyl group of a hydroxypropyl group. The introduction of the compound to be a linker into the cyclodextrin may or may not be carried out by hydroxyalkylating the hydroxyl group of the cyclodextrin with propylene oxide or the like, but is preferably carried out by hydroxyalkylating. In one embodiment of the present invention, the hydroxypropyl group introduced into the cyclodextrin is a 2-hydroxypropyl group. Depending on the linker to be introduced, the terminal of the hydroxypropyl group introduced into the hydroxyl group of the cyclodextrin can also be further modified.

The compound that can be used as a linker in the present invention is not particularly limited as long as it can bind a monovalent group derived from the above-mentioned boronic acid or its derivative to a cyclodextrin, but is preferably a compound having a reactive group at one end that is reactive with the hydroxyl group of the cyclodextrin and/or hydroxyl group of the hydroxypropyl group, and a reactive group at the other end that is reactive with the functional group of the above-mentioned boronic acid or its derivative. In addition, when the end of the hydroxypropyl group is further modified, a compound having a reactive group at one end that is reactive with the modified end and a reactive group at the other end that is reactive with the functional group of the above-mentioned boronic acid or its derivative is preferably used as a linker. Examples of the compound to be a linker include, although not limited to, compounds represented by the following formula (La). 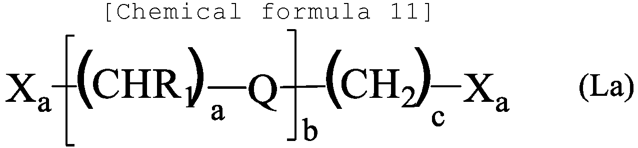 [wherein, R₁ each independently represents a hydrogen atom, a carboxy group which may be substituted with an alkyl group having 1 to 6 carbon atoms, a hydroxy group which may be substituted with an alkyl group having 1 to 6 carbon atoms, a mercapto group which may be substituted with an alkyl group having 1 to 6 carbon atoms, an aryl group having 6 to 14 carbon atoms, or an alkyl group having 1 to 6 carbon atoms;
Q each independently represents -O-, -S-, -NH-, - SO₂-, -CO-, -NHCO-, -CONH-, -OCO-, -COO-, -SCO-, -COS-, -NHCONH-, -NHCSNH-, -OCONH-, or -NHCOO-;
a each independently represents an integer of 1 to 6;
b represents an integer of 1 to 6;
c represents an integer of 1 to 6;
Xₐ each independently represents -OH, -SH, -NH₂, - SO₃H, -COOH, -NHCOOH, -CONH₂, -OCOOH, -COOH, -SCOOH, - COSH, -NHCONH₂, -NHCSNH₂, -OCONH₂, or -NHCOOH.].

Examples of the alkyl group having 1 to 6 carbon atoms include methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl, 2-ethylbutyl, cyclopropyl, cyclobutyl, cyclopentyl, and cyclohexyl.

Examples of the aryl group having 6 to 14 carbon atoms include phenyl, 1-naphthyl, 2-naphthyl, 1-anthryl, 2-anthryl, and 9-anthryl.

Preferred examples thereof include compounds represented by the following formula (Lb). 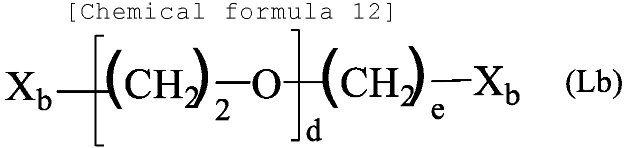 [wherein, d represents an integer of 1 to 6, e represents an integer of 1 to 3, and each X_{b} independently represents -OH, -SH, -NH₂, -SO₃H, -COOH, -NHCOOH, -CONH₂, -OCOOH, -COOH, -SCOOH, -COSH, - NHCONH₂, -NHCSNH₂, -OCONH₂, or -NHCOOH.]

The method for introducing the compound to be a linker into a cyclodextrin can be appropriately selected depending on, for example, the reactive group of the compound to be a linker to be reacted with the hydroxyl group of the cyclodextrin and/or the terminal group of the introduced hydroxypropyl group. In addition, the bonding of the monovalent group derived from the above-mentioned boronic acid or its derivative to the compound to be a linker introduced into the cyclodextrin can be appropriately selected depending on the reactive group of the compound to be a linker to be reacted with the reactive group of those groups.

Although not limited thereto, when the compound to be a linker has an amino group as a reactive group with the hydroxyl group of the hydroxypropyl group introduced into the cyclodextrin, for example, the compound to be a linker can be introduced into the cyclodextrin via an amide bond, for example, by using CDI as a catalyst. Also, when the reactive group at the other end of the compound to be a linker introduced into the cyclodextrin is an amino group, and when the boronic acid or its derivative has a carboxyl group, for example, by using NHS and EDC as catalysts to activate and react the carboxyl group with the amino group, the boronic acid or its derivative can be bound to the cyclodextrin via the linker by an amide bond.

The content of boronic acid in the boronic acid-containing modified polyrotaxane can be adjusted arbitrarily by changing the addition ratio of boronic acid or its derivative to a cyclodextrin. Here, the content of boronic acid or its derivative (content of boronic acid) can be expressed as either the ratio to one molecule of a polyrotaxane or the ratio to one molecule of a cyclodextrin. The content of boronic acid is preferably 0.1 to 5.0 per molecule of the cyclodextrin, more preferably 0.2 to 3.0, and even more preferably 0.5 to 2.0. Alternatively, the content of boronic acid is preferably 5 to 400 per molecule of the polyrotaxane, more preferably 10 to 200, and even more preferably 20 to 100.

### [Linear molecule]

The linear molecule of the modified polyrotaxane of the present invention is not particularly limited as long as it is a compound capable of penetrating the opening of a cyclodextrin and having a functional group reacting with a blocking group. Preferred linear molecules include polyalkylene glycols such as polyethylene glycol, polypropylene glycol and polytetramethylene glycol, hydroxyl-terminated polyolefins such as polybutadiene diol, polyisoprene diol, polyisobutylene diol, poly(acrylonitrile-butadiene) diol, hydrogenated polybutadiene diol, polyethylene diol and polypropylene diol, polycaprolactone diol, polyesters such as polylactic acid, polyethylene adipate, polybutylene adipate, polyethylene terephthalate and polybutylene terephthalate, or copolymers of these molecules. In terms of ease of synthesis of the polyrotaxane, polyethylene glycol, polypropylene glycol, and copolymers of polyethylene glycol and polypropylene glycol (e.g., PEG-PPG-PEG, etc.) are preferred.

Further examples of the linear molecule include terminal functional polysiloxanes such as terminal silanol type polydimethylsiloxane, terminal amino group chain polymers such as terminal amino group polyethylene glycol, terminal amino group polypropylene glycol and terminal amino group polybutadiene, terminal carboxyl group chain polymers such as terminal carboxyl group polyethylene glycol, terminal carboxyl group polypropylene glycol and terminal carboxyl group polybutadiene, and trifunctional or higher multifunctional chain polymers having three or more of the above-mentioned functional groups in one molecule, all of these have a functional group at the end. Among these, terminal amino group polyethylene glycol, terminal carboxyl group polyethylene glycol, terminal amino group polypropylene glycol, terminal carboxyl group polypropylene glycol, terminal amino group polypropylene glycol-polypropylene glycol copolymer, and terminal carboxyl group polypropylene glycol-polypropylene glycol copolymer are preferred.

The weight average molecular weight of the linear molecule mentioned above is 2 kDa or more, preferably 3 kDa or more, more preferably 5 kDa or more, and 200 kDa or less, preferably 100 kDa or less, more preferably 50 kDa or less. By having the molecular weight of the linear molecule in this range, the boronic acid-containing modified polyrotaxane can have a sufficient number of cyclodextrins and boronic acids and can have preferable solubility. Note that the weight average molecular weight is a value in terms of polystyrene measured by gel permeation chromatography (GPC).

In the boronic acid-containing modified polyrotaxane of the present invention, the number of cyclodextrin molecules included in the linear molecule (inclusion amount) is preferably 0.01 to 0.6, more preferably 0.05 to 0.5, even more preferably 0.1 to 0.4, and particularly preferably 0.2 to 0.3, assuming the maximum inclusion amount to be 1. By having the number of cyclodextrins within the above-mentioned range, the mobility and solubility of the cyclodextrin tend to be compatible. Note that the maximum inclusion amount of a cyclodextrin is a theoretical value determined by the molecular chain length of the linear molecule and the cavity size of the cyclodextrin.

The maximum inclusion amount of cyclodextrin molecules can be determined by the length of the linear molecule and the thickness of the cyclodextrin molecule in the chain direction of the linear molecule. For example, when the linear molecule is polyethylene glycol and the cyclodextrin molecule is α-cyclodextrin, the maximum inclusion amount is experimentally determined as described in Macromolecules 1993, 26, 5698-5703. For example, when 20 kDa polyethylene glycol is used as the linear molecule, the maximum inclusion amount is 227 α-cyclodextrin molecules.

### [Blocking Group]

The blocking groups of the modified polyrotaxane of the present invention may be any group as long as they are arranged at both ends of the linear molecule and a cyclodextrin does not leave from the linear molecule, and preferably include a bulky group or an ionic group, and among them, a bulky group is preferable. Only one type of such blocking group may be used, or multiple types may be used.

Examples of the blocking group include dinitrophenyl groups such as 2,4-dinitrophenyl group and 3,5-dinitrophenyl group, groups derived from cyclodextrin; an adamantyl group, an adamantylamino group, a trityl group, a fluorescenyl group, a pyrenyl group, substituted phenyl groups (substituents include, but are not limited to, alkyl groups, alkyloxy groups, hydroxyl group, halogens, cyano group, sulfonyl group, carboxy group, amino group, and phenyl group, and one or more substituents may be present), optionally substituted polycyclic aromatic groups (substituents include, but are not limited to, the same as those described above, and one or more substituents may be present), steroids, and the like.

Among these, from the viewpoint of ease of introduction of a blocking group, a dinitrophenyl group, groups derived from cyclodextrin; an adamantyl group, an adamantylamino group, a trityl group, a fluorescenyl group and a pyrenyl group are preferred, and an adamantyl group, an adamantylamino group and a trityl group are more preferred.

A compound having a moiety that serves as a blocking group and a group that reacts with the functional groups present at both ends of the linear molecule can be reacted with the linear molecule to introduce blocking groups to both ends of the linear molecule. The introduction can be carried out with reference to known methods, and for example, the method described in WO 2005-080469 can be used.

The weight-average molecular weight of the boronic acid-containing polyrotaxane of the present invention is preferably in the range of 10 to 1000 kDa, more preferably in the range of 20 to 500 kDa, and even more preferably in the range of 50 to 200 kDa. By having the weight-average molecular weight in this range, blood retention and tumor accumulation tend to be improved.

The boronic acid-containing polyrotaxane of the present invention can be produced, for example, by the following steps. The polyrotaxane can be produced by the steps of: mixing a cyclodextrin and a linear molecule, penetrating the opening of the cyclodextrin in a skewered manner with the linear molecule to include the linear molecule in the cyclodextrin to obtain a pseudopolyrotaxane; blocking both ends of the linear molecule of the pseudopolyrotaxane obtained with blocking groups to prevent the cyclodextrin from being released from the skewered state; hydroxypropylating a portion or all of the hydroxyl groups of the cyclodextrin in the polyrotaxane obtained with propylene oxide; reacting the hydroxyl group of the cyclodextrin and/or the hydroxyl group of the hydroxypropyl group with the compound as a linker to obtain a polyrotaxane having the compound to be a linker introduced therein; and reacting boronic acid or a derivative thereof with the compound to be a linker to obtain a boronic acid-containing modified polyrotaxane in which a monovalent group derived from boronic acid or a derivative thereof is bound to the cyclodextrin via the linker.

The boronic acid-containing polyrotaxane of the present invention can be converted into a boronic acid ester by binding a sugar to the boronic acid portion, thereby improving the solubility. The sugar can be appropriately selected from known sugars, and examples thereof include sugar alcohols such as sorbitol, mannitol, xylitol, erythritol, etc.; monosaccharides such as glucose, fructose, mannose, galactose, etc.; disaccharides such as sucrose, trehalose, lactose, maltose, etc.; or oligosaccharides, and preferably glucose, fructose, sucrose, etc. The sugar may be one type or multiple types.

### [Pharmaceutical Composition]

The boronic acid-containing modified polyrotaxane of the present invention has an anticancer effect by itself and thus can be used as an anticancer drug by itself, and also has a boron atom and thus can be used as a drug for BNCT as a boron drug using ¹⁰B. Therefore, a pharmaceutical composition containing the boronic acid-containing modified polyrotaxane of the present invention as an active ingredient is also a pharmaceutical composition that can be used for boron neutron capture therapy (BNTC), which utilizes the accumulation of the boronic acid-containing modified polyrotaxane in tumor cells and the selective uptake into cancer cells via sialic acid and further can produce the effect of destroying only cancer cells using α particles (⁴He particles) and ⁷Li particles of high-LET radiation generated by the reaction of boron (¹⁰B) with thermal neutrons.

The boronic acid-containing modified polyrotaxane of the present invention may be used as a drug for BNCT as it is, or may be formulated into various dosage forms by a method known to those skilled in the art using a pharmaceutically acceptable carrier or excipient. The carrier or excipient to be used is known to those skilled in the art and can be selected appropriately. Examples thereof include, but are not limited to, crosslinkers, dissolving agents, emulsifiers, moisturizers, cooling agents, inorganic powders, antioxidants, preservatives, colorants, flavoring agents, pH adjusters, and stabilizers. The pharmaceutical composition of the present invention can be manufactured using means and methods known to those skilled in the art. For example, when manufacturing an injection or infusion, a pharmaceutically acceptable carrier such as physiological saline or phosphate buffered saline can be used. When preparing the pharmaceutical composition of the present invention, pharmaceutically acceptable additives such as thickeners, absorption promoters, pH adjusters, preservatives, dispersants, wetting agents, stabilizers, preservatives, suspending agents, and surfactants may be used. In addition, since the boronic acid-containing modified polyrotaxane in the pharmaceutical composition of the present invention is also a carrier molecule, it can be used as it is for normal drug administration in radiation therapy, etc., without using other carrier molecules.

The dosage form of the pharmaceutical composition of the present invention is not particularly limited, and can be appropriately selected according to the site, size, type of the cancer to be treated, and condition of the patient. The drug of the present invention may be liquid, semi-solid, or solid. Examples of dosage forms of the drug of the present invention include, not limited to, injections, infusions, nasal drops, eye drops, sprays, lotions, creams, gels, ointments, powder coatings, liquid coatings, patches, other transdermal absorption agents, suppositories, tablets, capsules, powders, granules, syrups, aerosols, inhalants, transmucosal agents, etc. Alternatively, the drug of the present invention may be in the form of a lyophilized product that is suspended in a pharmaceutically acceptable carrier such as physiological saline or phosphate buffered saline at the time of administration. The pharmaceutical composition of the present invention is preferably in the form of liquid.

The administration route of the pharmaceutical composition of the present invention is not particularly limited, and can be appropriately selected according to the site, size, type of the cancer to be treated, the condition of the patient, etc. Examples of the administration route of the pharmaceutical composition of the present invention include systemic administration and local administration, and include, though not limited to, subcutaneous, intradermal, intramuscular, intravenous, infusion, intraarterial (e.g., via the carotid artery), intramedullary, intrathecal, intraventricular, intraperitoneal, or intranasal administration, oral administration, transmucosal administration, enteral administration, eye drops, ear drops, inhalation, transdermal administration, and intratumoral administration. Preferably, it is administered intravenously or subcutaneously.

The pharmaceutical composition of the present invention is administered to mammals (e.g., humans, cows, horses, pigs, dogs, cats, monkeys, mice, rats, particularly humans). The preferred administration form and formulation form are appropriately selected by a physician depending on the age, sex, constitution, symptoms, treatment time, etc. of the subject (patient). The dosage of the pharmaceutical composition of the present invention can be appropriately determined by a physician depending on the type, site, size and type of the cancer to be treated, and condition of the subject (patient). The dosage of the boronic acid-containing modified polyrotaxane of the present invention to a human is appropriately determined depending on the above-mentioned conditions of the subject. When the pharmaceutical composition of the present invention is used for BNCT, it is preferable to use an amount that can achieve the concentration of boron-10 in tumor tissue shown below.

When the pharmaceutical composition of the present invention is used for BNCT, the administration method may be any method that delivers the boronic acid-containing modified polyrotaxane to the vicinity of the tumor, but is preferably intravenous administration, intraperitoneal administration, or transdermal administration. When the pharmaceutical composition of the present invention is used for diagnosis in PET, the administration method is preferably intravenous administration.

When the boronic acid-containing polyrotaxane of the present invention is used for BNCT, it can be used, for example, but not limited to, as follows. When the boronic acid-containing polyrotaxane of the present invention is used for BNCT, it is preferable to use it as an intravenous infusion. Prior to irradiation, an injection solution containing the boronic acid-containing polyrotaxane of the present invention can be administered to a subject (patient or animal) in advance, adjusted so that boron-10 accumulates in the tumor, and then epithermal neutron radiation can be irradiated. Alternatively, prior to irradiation, an injection solution of the present invention can be administered to a subject in advance, adjusted so that boron-10 accumulates in the tumor, and then epithermal neutron radiation can be irradiated while continuing the administration. The dosage of the injection solution of the present invention is not particularly limited, but can be controlled to achieve a preferred intracellular boron concentration. Such a dosage is set depending on the type and progression of the tumor to be treated, the age and weight of the subject, etc., but when the injection solution of the present invention is used for intravenous administration, it is administered by drip infusion into a vein at a rate of about 200 to about 500 ml per hour for 1.5 to 4.0 hours, preferably 2.0 to 3.6 hours. The administration can be started before the start of neutron irradiation, or can be continuously administered from before the start of irradiation until the end of the irradiation.

The amount of the boronic acid-containing polyrotaxane administered in BNCT is not particularly limited as long as it can effectively damage (kill) tumor cells by neutron irradiation, and for example, it is desirable that the concentration of boron-10 in tumor tissue is about 10 ppm (about 50 boron-10 atoms per cell) to about 100 ppm or less, preferably about 20 ppm to about 60 ppm, and more preferably about 30 ppm to about 40 ppm.

It is preferable to control the neutron irradiation so that the nuclear reaction of epithermal neutrons efficiently occurs in the tumor cells, and the alpha rays and 7Li particles generated by the nuclear reaction can kill only the tumor cells. The radiation dose is calculated based on the boron concentration in blood and the neutron fluence irradiated to the tissue, and the X-ray equivalent dose can be calculated by multiplying the radiation dose by the effective relative biological effectiveness (RBE) of the boronic acid-containing polyrotaxane.

Prior to administration of the boronic acid-containing polyrotaxane of the present invention, positron emission tomography can be used to measure accumulation. For example, in addition to the boronic acid-containing polyrotaxane, a radioactive compound (18F-boronic acid-containing polyrotaxane) in which the boronic acid-containing polyrotaxane is labeled with the radionuclide 18F can be administered, and the accumulation of the boron compound can be estimated by imaging its whole-body distribution by a PET examination. Although not limited thereto, it is preferable to administer the compound to a subject in which the boron concentration ratio of cancer tissue/normal tissue is 3 or more in such a PET examination.

The cancers to which ¹⁰B drugs using the boronic acid-containing modified polyrotaxane of the present invention can be applied include, but are not limited to, lymphoma (Hodgkin's and non-Hodgkin's), carcinoma, carcinoma of solid tissue, squamous cell carcinoma, adenocarcinoma, sarcoma, glioma, high-grade glioma, blastoma, neuroblastoma, plasmacytoma, histiocytoma, melanoma, hypoxic tumor, myeloma, AIDS-related lymphoma or sarcoma, metastatic cancers, or cancers in general.

More specific examples thereof include lymphoma, B-cell lymphoma, T-cell lymphoma, mycosis fungoides, Hodgkin's disease, myeloid leukemia, bladder cancer, brain cancer, nervous system cancer, head and neck cancer, squamous cell carcinoma of the head and neck, kidney cancer, lung cancer (e.g., small cell lung cancer and non-small cell lung cancer), neuroblastoma/glioblastoma, ovarian cancer, pancreatic cancer, prostate cancer, skin cancer, liver cancer, melanoma; squamous cell carcinoma of the mouth, throat, larynx, and lung; colon cancer, cervical cancer, cervical carcinoma, breast cancer, and epithelial cancer, renal cancer, genitourinary cancer, lung cancer, esophageal carcinoma, gastric cancer, head and neck carcinoma, large bowel cancer, hematopoietic cancer; testicular cancer; colon and rectal cancer, or pancreatic cancer.

When the pharmaceutical composition of the present invention is used in BNCT, the pharmaceutical composition of the present invention is administered to a patient, and after a sufficient time has elapsed for the boronic acid-containing polyrotaxane of the present invention to reach the site to be treated, the site is irradiated with neutrons. In irradiating with neutrons, a nuclear reactor or accelerator-type neutron generator is used, and various conditions required for treatment, such as the neutron dose, neutron spectrum, and irradiation time, are determined.

The administration of the pharmaceutical composition of the present invention and neutron irradiation can be performed once or multiple times, but since the boronic acid-containing polyrotaxane of the present invention has excellent selectivity and accumulation in cancer cells, sufficient effects can be expected with fewer administrations and neutron irradiation, and the interval between administration and neutron irradiation can be shortened compared to conventional boron drugs. The actual number of administrations and neutron irradiations can be determined by a physician taking into consideration the site and type of the cancer, the degree of cancer shrinkage, the patient's condition, etc.

The pharmaceutical composition of the present invention can be used in combination with known chemotherapy, surgical therapy, radiation therapy, hyperthermia, immunotherapy, and the like.

Although not bound by this theory, in the boronic acid-containing modified polyrotaxane of the present invention, the cyclodextrin skewered on the linear molecule can move freely on the linear molecule, and therefore, when it reaches the vicinity of a target cell, the boronic acid molecule (e.g., a phenylboronic acid derivative) bound to the cyclodextrin can be presented while moving to the target molecule (e.g., sialic acid) expressed on the surface of the target cell, and it is expected that efficient multivalent interaction with the target molecule will be achieved, which may enable highly efficient tumor-selective delivery of boron drugs.

### [Examples]

The present invention will be specifically described below with reference to examples, but the present invention is not limited to the following examples.

An example using FPBA-PRX (fluorophenylboronic acid-rotaxane), which is one embodiment of the boronic acid-containing modified polyrotaxane of the present invention, will be described below.

### (Example 1: Preparation of FPBA-PRX)

FIG. 1 shows an example of the preparation route of FPBA-PRX. FPBA-PRX was prepared as follows. α-Cyclodextrin (α-CyD) was dissolved in water, and polyethylene glycol (PEG-NH₂) with aminated both ends was added, followed by stirring overnight at 4°C. After confirming the formation of a precipitate of polypseudorotaxane (PPRX), adamantane acetic acid and a condensing agent were added, followed by stirring for 48 hours. The product was washed with purified water, and unreacted α-CyD, PEG-NH₂ and adamantane acetic acid were removed to obtain a polyrotaxane (α-PRX) consisting of α-CyD.

Next, in order to improve the solubility of α-PRX, α-PRX was dissolved in an aqueous NaOH solution (1N), reacted with propylene oxide, dialyzed in water, and freeze-dried to obtain hydroxypropylated PRX (HP-PRX). The obtained HP-PRX was dissolved in DMSO, reacted with 1,2-Bis(2-aminoethoxy)ethane, dialyzed in water, then, freeze-dried to obtain N-[2-[2-(2-aminoethoxy)ethoxy]ethyl]amidated polyrotaxane (NH₂-PRX) .

4-Carboxy-3-fluorophenylboronic acid (FPBA) (109 mg) was dissolved in DMSO (100 mL), and NHS (106.3 mg) and EDC (187.5 mg) were added, followed by stirring at room temperature for 5 hours to activate the carboxy group of FPBA. NH₂-PRX (260 mg) was added to the activated FPBA, and triethylamine (136 µL) was added dropwise, followed by stirring at room temperature for 24 hours. Thereafter, unreacted FPBA was removed by dialysis (Spectra/Por (registered trademark) Membrane MWCO: 8 kDa, solvent: water), followed by lyophilization to obtain FPBA-PRX.

### (Example 2: Structural analysis of FPBA-PRX)

FPBA-PRX obtained in Example 1 was analyzed by ¹H-NMR spectrum. As a result, anomeric protons of α-CyD were observed around 4.9 ppm and 5.1 ppm, a peak derived from the ethoxy group of PEG was observed around 3.5 ppm, and a peak derived from CH of FPBA was observed around 7.3 to 7.6 ppm, confirming that FPBA-PRX was produced. Next, the number of α-CyDs in one molecule of FPBA-PRX was calculated from the peak area ratio of α-CyD to PEG, and was about 54.4. In addition, since theoretically, a maximum of about 227 α-CyDs can be penetrated into the PEG (20 kDa) used as the axis molecule, the penetration rate of FPBA-PRX was calculated to be about 24%. In addition, as a result of comparing the peak areas of α-CyD and FPBA, it was confirmed that 0.79 FPBAs were introduced per CyD molecule, and the number of FPBAs per molecule of FPBA-PRX was calculated to be about 43. The total molecular weight of FPBA-PRX calculated based on the above values was 107 kDa. The results are shown in the table below.

**[Table 1]**

| **Number of α-CyD** | **Coverage (%)** | **Number of FPBA** | **M.W. (kDa)** |
|---|---|---|---|
| **54.4** | **24** | **43** | **107** |

### (Comparative Example 1: Preparation of FPBA-C)

FPBA-C (fluorophenylboronic acid-cellulose) was prepared as a control with low mobility of FPBA. FIG. 2 shows the preparation route of FPBA-C. HPC (hydroxypropyl cellulose) was dissolved in DMSO and reacted with 1,2-Bis(2-aminoethoxy)ethane. After dialysis in water and lyophilization, N-[2-[2-(2-aminoethoxy)ethoxy]ethyl]amidated HPC (NH₂-C) was obtained. FPBA (24.5 mg) was dissolved in DMSO (50 mL), and NHS (21.6 mg) and EDC (38.2 mg) were added, followed by stirring at room temperature for 5 hours to activate the carboxy group of FPBA. NH₂-C (93.8 mg) was added to the activated FPBA, triethylamine (28 µL) was added dropwise, and the mixture was stirred at room temperature for 24 hours. After that, unreacted FPBA was removed by dialysis, and then freeze-dried to obtain FPBA-C.

### (Comparative Example 2: Analysis of FPBA-C)

FPBA-C obtained in Comparative Example 1 was subjected to ¹H-NMR spectrum analysis. As a result, a peak derived from the CH of cellulose was observed at around 4.3 ppm, and a peak derived from the CH of FPBA was observed at around 7.3 to 7.6 ppm, and it was confirmed that FPBA-C was produced. Next, the peak areas of cellulose and FPBA were compared, and it was resultantly confirmed that about 35 FPBAs were introduced per molecule of cellulose. The number of FBPAs bound to one molecule of cellulose was 35, which is considered to be roughly equivalent to the 43 FBPAs bound to one molecule of the rotaxane in Example 1, and therefore it was found to be suitable as a comparative example. Furthermore, it was confirmed that the total molecular weight of FPBA-C was about 116 kDa, and the chemical properties of FPBA-PRX and FPBA-C were roughly equivalent.

### (Example 3: Evaluation of physical properties of FPBA-PRX: particle size and ζ-potential)

In order to demonstrate the EPR effect, it is generally considered important that the particle size of the formulation is 200 nm or less and that the charge is neutral. The physicochemical properties of FPBA-PRX and FPBA-C prepared in Example 1 and Comparative Example 1 were evaluated. FPBA-PRX (0.53 mg/mL) or FPBA-C (0.58 mg/mL) was added to a 5% mannitol aqueous solution (1 mL, pH 7.4), and after pipetting, the particle size and ζ-potential were measured using a Malvern Zetasizer Nano-ZS. The measurement results are shown in the following table. Each value represents the mean value ± SEM of three measurements.

**[Table 2]**

| **Sample** | **Mean diameter (nm)** | **ζ-Potential (mV)** |
|---|---|---|
| **FPBA-PRX** | **14.53±0.95** | **-2.019±1.83** |
| **FPBA-C** | **11.98±3.72** | **0.052±0.02** |

The particle size of FPBA-PRX was about 15 nm, and that of FPBA-C was about 12 nm. In addition, it was suggested that the ζ-potentials of FPBA-PRX and FPBA-C were both near neutral. From these results, it was confirmed that FPBA-PRX and FPBA-C have good particle properties as materials for drug delivery systems (DDS). Therefore, it was confirmed that FPBA-PRX may be a particle that can be expected to be used not only for active targeting targeting sialic acid (SA) but also for passive targeting utilizing the EPR effect.

### (Example 4: Evaluation of physical properties of FPBA-PRX: Confirmation of interaction with SA)

PBA is known to reversibly bind to 1,2-and 1,3-diols of sugars via boronic acid, and has been reported to interact with SA. To examine whether FPBA-PRX interacts with SA, evaluation was performed using Alizarin Red S (ARS). ARS emits fluorescence by specifically binding to phenylboronic acid. FPBA-PRX solution (12 µM), ARS solution (500 µM), and SA solution (0, 20, 30, 40 mM) were prepared in PBS, and the FPBA-PRX solution (500 µL) was mixed with SA solutions of various concentrations (500 µL) and allowed to stand at room temperature for 10 minutes. Then, FPBA-PRX/SA mixed solutions of various concentrations (500 µL) were added to the ARS solution (500 µL) and allowed to stand at room temperature for 10 minutes. Thereafter, the fluorescence derived from ARS was measured using a fluorescence plate reader. The results are shown in FIG. 3. As shown in the figure, strong fluorescence was observed by adding FPBA-PRX to ARS. Furthermore, when various concentrations of SA were added, the fluorescence intensity derived from ARS decreased depending on the concentration of SA added. These results suggest that FPBA-PRX interacts with SA.

In addition, the dissociation constants of FPBA-PRX and FPBA-C, which is a control, with SA were evaluated using the quartz crystal microbalance method (QCM), and it was confirmed that FPBA-PRX interacts more strongly with SA than FPBA-C.

### (Example 5: Evaluation of cytotoxicity of FPBA-PRX)

The cytotoxicity of FPBA-PRX was evaluated using HeLa cells as an index of safety evaluation. HeLa cells were seeded in a 96-well culture plate at 1.5 × 10⁴ cells/well and cultured in a 10% FBS-containing medium for 24 hours. After washing the cells twice with serum-free medium, serum-free medium (100 µL) containing FPBA-PRX (0-25 µM) was added and treated for 4 hours. After washing twice with HBSS, HBSS (100 µL) and WST-8 solution (10 µL) were added, and the cells were incubated at 37°C for 1 hour, after which the absorbance was measured using a microplate reader. In addition, the cell viability at the time of sample treatment was calculated by setting the absorbance of serum-free medium containing Tween 20 (1%) and the sample-free system as 0% and 100%, respectively. As a result, FPBA-PRX maintained nearly 100% viability at all concentrations, confirming that it does not exhibit cytotoxicity to HeLa cells by itself. In addition, the boron concentration in FPBA-PRX (25 µM), the maximum concentration measured this time, was 1.22mM, which is roughly equivalent to the boron concentration in BPA, which is widely used in clinical practice, demonstrating the possibility that FPBA-PRX has biocompatibility that allows clinical application.

### (Example 6: Evaluation of intracellular uptake of FPBA-PRX)

It is known that SA is present at the non-reducing end of the sugar chain on the cell surface, plays an important role in cell recognition, and is highly expressed in cancer cells compared to normal cells. TRITC-FPBA-PRX and TRITC-FPBA-C, which were labeled with the fluorescent substance TRITC, and TRITC-HP-PRX, which was obtained by labeling PRX (HP-PRX) not modified with FPBA with TRITC, were prepared, and their intracellular uptake in various cancer cells and normal cells was examined by flow cytometry.

NH₂-PRX (50 mg) was dissolved in DMSO (20 mL), and a TRITC solution (5 mg/mL, 200 µL) was added, followed by stirring at room temperature for 24 hours to prepare TRITC-NH₂-PRX. Next, FPBA (23 mg) was dissolved in DMSO (20 mL), and NHS (20.5 mg) and EDC (36 mg) were added, followed by stirring at room temperature for 5 hours to activate the carboxy group of FPBA. Activated FPBA was added to TRITC -NH₂-PRX, and triethylamine (26 µL) was added dropwise, followed by stirring at room temperature for 24 hours. Unreacted FPBA and TRITC were removed by dialysis, followed by freeze-drying to prepare TRITC-FPBA-PRX.

TRITC-FPBA-C was prepared in the same manner using NH₂-C (55 mg). TRITC-HP-PRX was prepared by reacting TRITC in the same manner using NH₂-PRX (50 mg), removing unreacted TRITC by dialysis, and then lyophilizing the mixture.

Intracellular uptake was measured as follows. HeLa cells were seeded in a 24-well culture plate at 3.75 × 10⁴ cells/well and cultured in a medium containing 10% FBS for 24 hours. After washing the cells twice with serum-free medium, serum-free medium (500 µL) containing TRITC-FPBA-PRX, TRITC-FPBA-C, TRITC-HP-PRX (0, 50, 100, 200 nM: when adjusted at each polymer concentration) or serum-free medium (500 µL) containing TRITC-FPBA-PRX, TRITC-FPBA-C (0, 2, 4, 8 µM: when adjusted at FPBA concentration in the polymer) was added, and the cells were treated for 4 hours. After washing once with HBSS, the cells were detached from the plate by trypsin-EDTA method, the supernatant was removed by centrifugation, and then HBSS (500 µL) containing 10% FBS was added to prepare a cell suspension, after which TRITC fluorescence was measured by flow cytometry. The amount of TRITC modification in each sample was corrected based on the TRITC-FPBA-PRX system.

Colon-26 cells were seeded at 1.0 × 10⁵ cells/well and cultured in 10% FBS-containing medium for 24 hours. After washing the cells twice with serum-free medium, serum-free medium (500 µL) containing TRITC-FPBA-PRX, TRITC-FPBA-C and TRITC-HP-PRX (0, 100, 200, 500 nM: when adjusted at each polymer concentration) or serum-free medium (500 µL) containing TRITC-FPBA-PRX and TRITC-FPBA-C (0, 5, 10, 20 µM: when adjusted at FPBA concentration in polymer) was added and treated for 4 hours. After that, the cells were washed once with HBSS, detached from the plate, and fluorescence was measured in the same manner.

The results for HeLa cells are shown in FIG. 4, and the results for Colon-26 cells are shown in FIG. 5. In FIG. 4A and FIG. 5A, the polymer concentrations contained in TRITC-FPBA-PRX, TRITC-HP-PRX, and TRITC-FPBA-C were kept constant, and in FIG. 4B and FIG. 5B, the FPBA concentrations were kept constant, and the uptake ability into various cancer cells was evaluated. As shown in FIG. 4, it was confirmed that TRITC-HP-PRX not modified with FPBA and TRITC-FPBA-C, whose ligand is low mobile, were hardly taken up into HeLa cells, whereas TRITC-FPBA-PRX was efficiently and concentration-dependently taken up. In addition, the same tendency was confirmed with the FPBA concentration kept constant, as shown in FIG. 4B. Furthermore, as shown in FIG. 5, it was confirmed that TRITC-FPBA-PRX was taken up into cells more efficiently than TRITC-HP-PRX and TRITC-FPBA-C also in Colon-26 cells. These results indicate that the dynamic properties of FPBA modifying PRX are important for highly efficient cellular uptake.

It is believed that FPBA-PRX is taken up into cells via SA, which is highly expressed on the surface of cancer cells, and is not taken up into normal cells. Next, to evaluate the safety of FPBA-PRX, the cellular uptake of FPBA-PRX was evaluated by flow cytometry in human normal kidney cells (HK2 cells), which are generally considered to have low expression of SA.

HK2 cells were seeded at 5.0 × 10⁴ cells/well and cultured in 10% FBS-containing medium for 24 hours. After washing the cells twice with serum-free medium, serum-free medium (500 µL) containing TRITC-FPBA-PRX, TRITC-HP-PRX (0, 200 nM) was added and treated for 4 hours. After washing once with HBSS, the cells were detached from the plate, centrifuged to remove the supernatant, and HBSS (500 µL) containing 10% FBS was added to prepare a cell suspension, and TRITC fluorescence was measured using a flow cytometer.

The results are shown in FIG. 6. From the figure, it was confirmed that TRITC-FPBA-PRX was hardly taken up by HK2 cells, and the amount taken up was almost the same as that of HP-PRX. From these results, it was confirmed that TRITC-FPBA-PRX generates dynamic multivalent interactions with SA due to its conversion to PRX, and is taken up more actively into various cancer cells that highly express SA compared to low-mobility polymers.

### (Example 7: Evaluation of pharmacokinetics of FPBA-PRX)

FPBA-PRX was administered intravenously to healthy mice, and blood retention was confirmed as follows. 5% mannitol solution (100 µL) containing TRITC-FPBA-PRX fluorescently labeled with TRITC was administered into the tail vein of mice (BALB/c). The dose was 5.8 mg/kg. After 5, 10, 30, 60, 180, and 360 minutes, blood (200 µL) was collected from the abdominal inferior vena cava under isoflurane anesthesia. The blood collected from the mice was centrifuged, and plasma (100 µL) was separated and the fluorescence of TRITC was measured using a fluorescent plate reader. In addition, TRITC-FPBA-PRX, TRITC-FPBA-C were dissolved in plasma and serially diluted to prepare a calibration curve, and the blood concentration was calculated from the fluorescence intensity to evaluate the blood retention of FPBA-PRX. The results of the blood concentration after intravenous administration of TRITC-FPBA-PRX are shown in FIG. 7. It was confirmed that about 12% of the administered amount of TRITC-FPBA-PRX remained in the blood 60 minutes after administration, and about 9% of the administered amount remained even 360 minutes after administration.

### (Example 8: Evaluation of tumor accumulation of FPBA-PRX)

As shown in Example 7, when FPBA-PRX was intravenously administered to healthy mice, approximately 88% of the administered dose was found to disappear from the blood 60 minutes after administration, then, FPBA-PRX was intravenously administered to cancer-bearing mice, and tumor accumulation was evaluated as follows. In order to clarify the tumor accumulation after intravenous administration of FPBA-PRX, a single dose of TRITC-FPBA-PRX or TRITC-FPBA-C was administered into the tail vein of a cancer-bearing mouse transplanted with Colon-26 cells, and the tumor was then collected and evaluated by measuring the fluorescence intensity of TRITC. A Colon-26 cell suspension (5 × 10⁵ cells/100 µL) was inoculated into the hind leg of a BALB/c male mouse (4 weeks old, 20 g) to prepare a mouse with Colon-26 cells transplanted subcutaneously. After about one week, mice whose tumors had reached a major axis of 8 mm were used in the experiment.

TRITC-FPBA-PRX or TRITC-FPBA-C-containing 5% mannitol solution (100 µL) was administered into the tail vein of the mouse. The dose was 5.8 mg/kg. After 30, 180 and 360 minutes, perfusion was performed under isoflurane anesthesia, and the tumor and each organ (heart, lung, spleen, liver, kidney) were excised. The excised tumor and organ were dissolved in a cell lysis solution and homogenized. Then, centrifugation was performed, the supernatant was separated, and after further centrifugation, the supernatant was separated, and the fluorescence of TRITC was measured with a fluorescent plate reader. In addition, TRITC-FPBA-PRX or TRITC-FPBA-C was dissolved in the lysate of the tumor and each organ, a calibration curve was created by serial dilution, and the concentration in the tumor and each organ was calculated from the fluorescence intensity.

The results of tumor accumulation rate after single administration of TRITC-FPBA-PRX or TRITC-FPBA-C into the tail vein are shown in FIG. 8. About 10% of the administered dose of TRITC-FPBA-PRX accumulated in the tumor 180 minutes after administration, and about 15% of the administered dose accumulated in the tumor 360 minutes after administration. Furthermore, it was confirmed that TRITC-FPBA-PRX showed a higher tumor accumulation rate than TRITC-FPBA-C.

Although not bound by this theory, it can be considered as follows. FPBA-C is expected to show the EPR effect as a polymer, and about 5% accumulation was confirmed due to multivalent interaction with SA, but FPBA-PRX was confirmed to accumulate in tumors more efficiently than FPBA-C, and showed very remarkable accumulation. As a factor of FPBA-PRX's high tumor accumulation, as shown in Table 2, it is considered that FPBA-PRX has physical properties suitable for the EPR effect, and in addition, dynamic multivalent interaction by FPBA functioned. It was also confirmed that FPBA-PRX interacts with SA with a higher association constant than FPBA-C. From the above, it is considered that FPBA-PRX accumulates in tumor tissues due to the EPR effect after intravenous administration, and presents FPBA on demand in accordance with the distribution of SA on the cell surface, and as a result, is efficiently taken up into tumor tissues via SA, and further shows excellent tumor accumulation by avoiding excretion by antiporters.

### (Example 9: Evaluation of tissue distribution of FPBA-PRX)

The tissue migration of TRITC-FPBA-PRX to various tissues excised in Example 8 was evaluated using the fluorescence intensity of TRITC as an index. The results are shown in FIG. 9. As shown in the figure, it was confirmed that TRITC-FPBA-PRX accumulated most in the tumor 3 hours after intravenous administration, and accumulated in the tumor more than in other organs at least until 6 hours after administration. On the other hand, it was confirmed that TRITC-FPBA-C accumulated mainly in the liver, kidney and spleen, although it accumulated partially in tumors. It is generally known that high molecular weight polymers are phagocytosed by the reticuloendothelial system (RES) after intravenous administration. In fact, since FPBA-C has a lower tumor selectivity than FPBA-PRX, it is considered that it was phagocytosed by the RES and accumulated in the liver and spleen in a time-dependent manner. From these facts, it was confirmed that FPBA-PRX migrated quickly to the tumor after intravenous administration, avoided phagocytosis by the RES, and was taken up into cancer cells via SA.

### (Example 10: Evaluation of T/B ratio)

In boron neutron capture therapy, it is important to evaluate the tumor/blood ratio (T/B ratio) and tumor/normal tissue ratio (T/N ratio) of boron drugs after intravenous administration from the viewpoint of safety in neutron irradiation. As in Example 8, TRITC-FPBA-PRX or TRITC-FPBA-C was administered once (5.8 mg/kg) into the vein of tumor-bearing mice, and the T/B ratio was measured after 3, 6, and 24 hours. The results are shown in FIG. 10. The T/B ratio of TRITC-FPBA-PRX was about 3 or more 24 hours after administration, and was higher than that of TRITC-FPBA-C at all times. According to the BNCT irradiation system development guidelines, from the standpoint of safety, BNCT requires that the T/B ratio and T/N ratio be 3 or more, while the FPBA-PRX of the present invention satisfies this requirement, and it has been confirmed that it has excellent cancer selectivity.

### (Example 11: Evaluation of safety of FPBA-PRX)

To evaluate the safety of FPBA-PRX, FPBA-PRX was administered to mice and blood biochemistry test values were measured. A single dose of 5% mannitol solution (100 µL) containing TRITC-FPBA-PRX (5.8 mg/kg) was administered into the tail vein of male BALB/c mice (4 weeks old, 20 g). Twenty four hours after administration, blood was collected from the abdominal inferior vena cava under isoflurane anesthesia. The blood collected from the mice was centrifuged and plasma was separated. Various blood biochemistry test values were quantified using a fully automated hematology analyzer. Physiological saline (Saline) was administered as a control. The measurement results are shown in the following table. Each value represents the mean value ± SEM of each measurement value of four mice. CRE: creatinine; BUN: blood urea nitrogen; AST: aspartate aminotransferase; ALT: alanine aminotransferase; LDH: lactate dehydrogenase.

**[Table 3]**

| **Sample** | **CRE (mg/dL)** | **BUN (mg/dL)** | **AST (U/L)** | **ALT (U/L)** | **LDH (mg/dL)** |
|---|---|---|---|---|---|
| **Saline** | **0.15±0.02** | **21.8±2.5** | **66±16** | **25±1.8** | **288±82** |
| **FPBA-PRX** | **0.14±0.01** | **26.4±2.6** | **76±19** | **30±3.0** | **298±105** |

As shown in the table, FPBA-PRX showed values equivalent to those of the saline group in all test items, confirming the possibility that FPBA-PRX is excellent in safety in vivo.

### (Example 12: Evaluation of folate-modified β-CyD-bound boronic acid-containing modified polyrotaxane)

It is known that folate receptor molecules are overexpressed on the surface of cancer cells, and it has been reported that folate may be a ligand for a cancer cell targeting drug system. In addition, folate-modified β-cyclodextrin, in which folate is bound as a ligand, has been reported for the purpose of DDS to cancer cells.

Folate-modified β-cyclodextrin (FA7-β-CyD) was bound to TRITC-FPBA-PRX prepared in the same manner as in Example 6 to prepare a folate-modified β-CyD-bound boronic acid-containing modified polyrotaxane, and the uptake into cells was evaluated. As shown in FIG. 11, the folate-modified β-CyD is considered to bind to the blocking group, adamantane.

Colon-26 cells were seeded at 1.0 × 10⁵ cells/well and cultured for 24 hours in a 10% FBS-containing medium. The cells were washed twice with serum-free medium, and then cultured for 1 hour with folate-containing medium (0.5-2 mM) added. Next, a medium containing TRITC-FPBA-PRX and FA7-β-CyD (TRITC-FPBA-PRX/FA7-β-CyD-containing medium) was added and cultured for 4 hours. Thereafter, the cells were washed once with HBSS, detached from the plate, then, the supernatant was removed by centrifugation, then, HBSS containing 10% FBS was added to prepare a cell suspension, and then the fluorescence of TRITC was measured with a flow cytometer. In order to confirm the effect of FA7-β-CyD, a medium containing TRITC-FPBA-PRX/FA7-β-CyD and further containing folate added was used. The results are shown in FIG. 12. It was confirmed that the addition of FA7-β-CyD increased the uptake of TRITC-FPBA-PRX into cells, and that this effect was inhibited in a concentration-dependent manner by adding folate to the medium. This confirmed that the binding of folate-modified cyclodextrin to TRITC-FPBA-PRX enhanced the cellular accumulation of FPBA-PRX.

### (Example 13: Evaluation of PEG dopamine-modified boronic acid-containing modified polyrotaxane)

It has been reported that binding PEG to a molecule improves blood retention, is expected to have the effect of avoiding recognition by normal tissues, and improves targeting to cancer cells.

PEG-dopamine-bound boronic acid-containing modified polyrotaxane was prepared by binding PEG-dopamine to FPBA-PRX prepared in the same manner as in Example 6, and the uptake into cells was evaluated. As shown in FIG. 11, it is believed that the diol moiety of PEG-dopamine and boronic acid reversibly bind. Such a bond is maintained at a near-neutral pH in blood, but is believed to dissociate at an acidic pH in the vicinity of cancer cells.

HeLa cells were seeded at 5.0 × 10⁴ cells/well and cultured for 24 hours in a medium containing 10% FBS. After washing the cells with serum-free medium, a mixed solution of TRITC-FPBA-PRX and PEG dopamine (TRITC-FPBA-PRX:PEG dopamine concentration ratios = 1:1, 1:2, and 1:4) was added and cultured for 4 hours. Thereafter, the cells were washed with HBSS, detached from the plate by the trypsin-EDTA method, then, the supernatant was removed by centrifugation, then, HBSS (500 µL) containing 10% FBS was added to prepare a cell suspension, and then the fluorescence of TRITC was measured by flow cytometry. The results are shown in FIG. 13. It is presumed that as the relative ratio of PEG dopamine increases, the proportion of boronic acid bound to PEG dopamine increases, and as a result, the uptake into cells targeting SA decreased.

Next, a cellular uptake test was performed at pH 7.4 and pH 6.5, and the cellular uptake was significantly increased at pH 6.5 compared to pH 7.4. The results are shown in FIG. 14. This suggests that as the pH becomes more acidic, the bond between boronic acid and PFG dopamine dissociates, increasing the cellular uptake.

### (Example 14: Evaluation of cytotoxicity to cancer cells)

Using the FPBA-PRX prepared in Example 1, the cytotoxicity against cancer cells was evaluated as follows. HeLa cells were seeded at 5.0 × 10⁴ cells/well and cultured in a medium containing 10% FBS for 24 hours. After removing the medium and washing with PBS, trypsin 2.5 mg/mL and FPBA-PRX (12 mg/mL)-containing DMEM medium were added (500 µL) and cultured for 3 hours. After washing twice with PBS, HBSS (100 µL) and WST-8 solution (10 µL) were added, and the mixture was incubated at 37°C for 30 minutes, and then the absorbance was measured using a microplate reader. The results are shown in FIG. 15. It was confirmed that FPBA-PRX exhibits cytotoxicity against cancer cells.

### (Example 15: Evaluation of antitumor activity)

Colon26 cells were transplanted into the right thigh of BALB/c mice (9 weeks old, male, n = 4-5 per group), and after the transplantation, the mice were raised until the tumor diameter reached about 7-9 mm. The mice were weighed and tumor size was measured on the day before irradiation, and the mice were divided into groups based on tumor volume. Group A: control; Group B: FPBA-PRX administration group, Group C: neutron irradiation group, Group D: FPBA-PRX administration + neutron irradiation group. In the FPBA-PRX administration groups (Group B, Group D), FPBA-PRX was administered at a dose of 500 mg/kg (administered at a concentration of 100 mg/mL) via the tail vein 6 hours before irradiation. This is equivalent to 10 mg/kg when converted to the ¹⁰B concentration. The administration time was 6 hours.

In the neutron-irradiated groups (groups C and D), an anesthetic was administered intraperitoneally, and the mice were then placed in an irradiation container and subjected to neutral irradiation (4800 mC, 40 min), and after the irradiation was completed, an antagonist was administered intraperitoneally and awakening was confirmed. The results are shown in FIG. 16. The figure shows the average tumor size of the nonirradiated group and the average tumor size of the irradiated group, respectively. The FPBA-PRX-administered + neutron irradiation group showed tumor suppression compared to the neutron irradiation group of the subject.

The above detailed description is merely illustrative of the objects and subject matter of the present invention and is not intended to limit the scope of the appended claims. Various modifications and substitutions to the described embodiments will be apparent to those skilled in the art from the teachings set forth in the present specification without departing from the scope of the appended claims.

This application is based on Japanese Patent Application No. 2022-46218 (filing date: March 23, 2022) filed in Japan, and the contents of which are incorporated in their entirety in the present specification.

### [Industrial Applicability]

The present invention provides a novel compound, boronic acid-containing modified polyrotaxane. The boronic acid-containing modified polyrotaxane of the present invention has excellent blood retention and tumor selectivity, and can be a component for a novel boron drug, thus, is useful.

## Claims

1. A boronic acid-containing modified polyrotaxane comprising a plurality of cyclodextrin molecules, a linear molecule that pierces the openings of the plurality of cyclodextrin molecules in a skewered manner, and blocking groups that are positioned at both ends of the linear molecule and prevent separation of the cyclodextrin molecule from the linear molecule, wherein at least a portion of the cyclodextrin molecules are bonded via a linker to a monovalent group derived from boronic acid or a derivative thereof.

2. The boronic acid-containing modified polyrotaxane according to claim 1, wherein the monovalent group derived from boronic acid or a derivative thereof is a monovalent group derived from a phenylboronic acid derivative or a pyridineboronic acid derivative.

3. The boronic acid-containing modified polyrotaxane according to claim 2, wherein the monovalent group derived from boronic acid or a derivative thereof is a group represented by the following formula (B1): 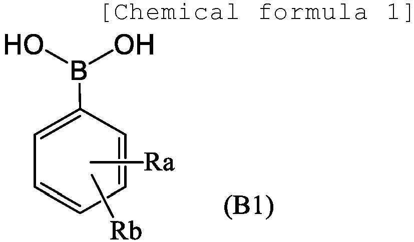 (wherein Ra represents a hydrogen atom or a halogen atom, and Rb represents -(CH₂)n-CO-*, -(CH₂)n-NH-*, - (CH₂)n-CH(NHR)CO-*, or -(CH₂)n-CH(C(O)OR)NH-* (wherein * represents a bond to a linker, n represents 0, 1, or 2, and R represents a hydrogen atom or a C1-C5 hydrocarbon group which may have a substituent)), or the following formula (B2): 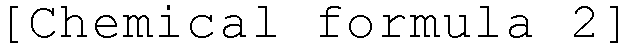 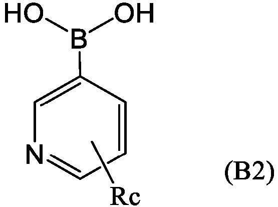 (wherein Rc represents -(CH₂)n-CO-*, -(CH₂)n-NH-*, - (CH₂)n-CH(NHR)CO-*, or -(CH₂)n-CH(C(O)OR)NH-* (wherein * represents a bond to a linker, n represents 0, 1, or 2, and R represents a hydrogen atom or a C1-C5 hydrocarbon group which may have a substituent)).

4. The boronic acid-containing modified polyrotaxane according to claim 3, wherein the monovalent group derived from boronic acid or a derivative thereof is a group represented by the following formula (B3): 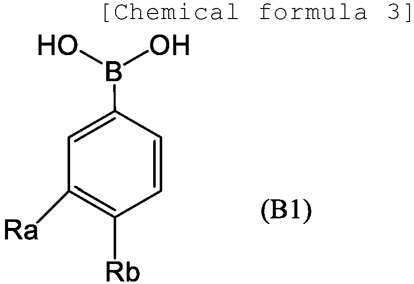 (wherein Ra represents a hydrogen atom or a halogen atom, and Rb represents -(CH₂)n-CO-*, -(CH₂)n-NH-*, - (CH₂)n-CH(NH₂)CO-*, or -(CH₂)n-CH(C(O)OH)NH-* (wherein * represents a bond to a linker, and n represents 0 or 1)), or the following formula (B4): 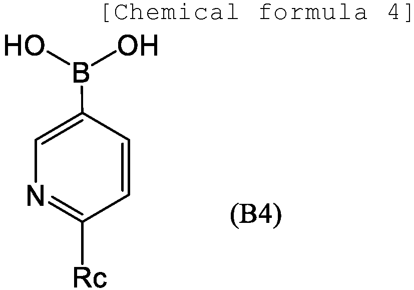 (wherein Rc represents -(CH₂)n-CO-*, -(CH₂) n-NH-*, - (CH₂)n-CH(NH₂)CO-*, or -(CH₂)n-CH(C(O)OH)NH-* (wherein * represents a bond to a linker, and n represents 0 or 1)).

5. The boronic acid-containing modified polyrotaxane according to claim 4, wherein the monovalent group derived from boronic acid or a derivative thereof is a monovalent group derived from a boronic acid derivative selected from the group consisting of 4-carboxyl-3-fluorophenylboronic acid, 6-carboxypyridine-3-boronic acid (5-boronopicolinic acid), 4-carboxyphenylboronic acid, p-boronophenylalanine, 4-amino-3-fluorophenylboronic acid, and 5-aminopyridine-3-boronic acid.

6. The boronic acid-containing modified polyrotaxane according to any one of claims 1 to 5, wherein the linear molecule is polyethylene glycol, polypropylene glycol, or a copolymer of polyethylene glycol and polypropylene glycol.

7. The boronic acid-containing modified polyrotaxane according to any one of claims 1 to 6, wherein the blocking group is an adamantyl group.

8. The boronic acid-containing modified polyrotaxane according to any one of claims 1 to 7, wherein the plurality of cyclodextrin molecules are a plurality of α-cyclodextrin molecules.

9. The boronic acid-containing modified polyrotaxane according to any one of claims 1 to 8, wherein the linker is represented by the formula (L1): 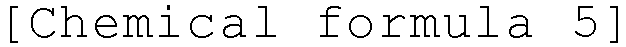 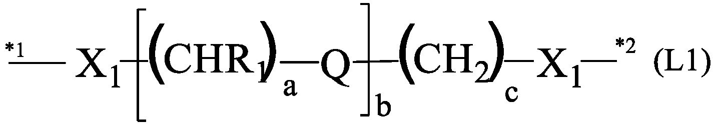 [wherein, *¹ is a site bonding to a cyclodextrin molecule, *² is a site bonding to a group containing boronic acid,
R₁ each independently represents a hydrogen atom, a carboxy group which may be substituted with an alkyl group having 1 to 6 carbon atoms, a hydroxy group which may be substituted with an alkyl group having 1 to 6 carbon atoms, a mercapto group which may be substituted with an alkyl group having 1 to 6 carbon atoms, an aryl group having 6 to 14 carbon atoms, or an alkyl group having 1 to 6 carbon atoms;
Q each independently represents -O-, -S-, -NH-, - SO₂-, -CO-, -NHCO-, -CONH-, -OCO-, -COO-, -SCO-, -COS-, -NHCONH-, -NHCSNH-, -OCONH-, or -NHCOO-;
a each independently represents an integer of 1 to 6;
b represents an integer of 1 to 6;
c represents an integer of 1 to 6;
X₁ each independently represents -O-, -S-, -NH-, - SO₂-, -CO-, -NHCO-, -CONH-, -OCO-, -COO-, -SCO-, -COS-, -NHCONH-, -NHCSNH-, -OCONH-, or -NHCOO-].

10. The boronic acid-containing modified polyrotaxane according to claim 9, wherein the linker is represented by the formula (L2): 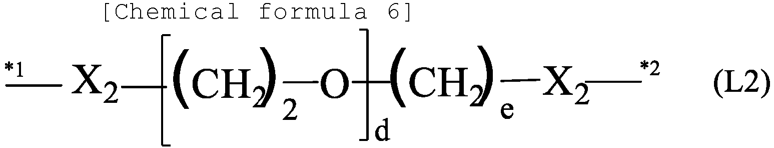 [wherein, *¹ is a site bonding to a cyclodextrin molecule, *² is a site bonding to a group containing boronic acid,
d represents an integer of 1 to 6;
e represents an integer of 1 to 3;
X₂ each independently represents -O-, -S-, -NH-,-SO₂-, -CO-, -NHCO-, -CONH-, -OCO-, -COO-, -SCO-, -COS-, -NHCONH-, -NHCSNH-, -OCONH-, or -NHCOO-].

11. The boronic acid-containing modified polyrotaxane according to any one of claims 1 to 10, wherein in the polyrotaxane in which the blocking group is an adamantyl group, folate-modified β-cyclodextrin is further bonded to the adamantyl group.

12. The boronic acid-containing modified polyrotaxane according to claim 11, wherein the plurality of cyclodextrin molecules are α-cyclodextrin molecules and the linear molecule is polyethylene glycol.

13. The boronic acid-containing modified polyrotaxane according to any one of claims 1 to 12, wherein polyethylene glycol-modified dopamine is further bonded to the boronic acid.

14. A pharmaceutical composition for use in boron neutron capture therapy for tumor diseases, comprising the boronic acid-containing modified polyrotaxane according to any one of claims 1 to 13 in which the boron in the boronic acid is ¹⁰B.

15. A method for treating a tumor disease by boron neutron capture therapy, comprising administering to a mammal a required amount of the boronic acid-containing modified polyrotaxane according to any one of claims 1 to 13 in which the boron in the boronic acid is ¹⁰B.
